# EUROPEAN PATENT SPECIFICATION

(11) **EP 3 135 341 B1**
(45) Date of publication and mention of the grant of the patent: **20.02.2019**
(21) Application number: 15182742.5
(22) Date of filing: 27.08.2015
(51) Int. Cl.: A61Q 19/02, A61K 8/9789

(54) **COSMETIC SKIN WHITENING COMPOSITIONS CONTAINING EXTRACTS DERIVED FROM IN VITRO PROPAGATED HYPERICUM HIRSUTUM**
KOSMETISCHE DEPIGMENTIERUNGSMITTEL MIT EXTRAKTEN AUS IN-VITRO PROPAGIERTEM HYPERICUM HIRSUTUM
COMPOSITIONS COSMÉTIQUES DE BLANCHIMENT DE LA PEAU CONTENANT DES EXTRAITS DÉRIVÉS DE PROPAGATION IN VITRO D'HYPERICUM HIRSUTUM

(43) Date of publication of application: 01.03.2017
(73) Proprietor: Latvijas Universitate, 1586 Riga (LV)
(72) Inventor: Boroduskis, Martins, LV-5232 Salas Region (LV); Ramata-Stunda, Anna, LV-1011 Riga (LV); Boroduske, Anete, Riga (LV); Nakurte, Ilva, LV-1058 Riga (LV); Kaktina, Elza, LV-1013 Riga (LV); Silamikele, Baiba, LV-1030 Riga (LV); Tomsone, Signe, LV-1006 Riga (LV); Rostoks, Nils, LV-1048 Riga (LV); Ancans, Janis, LV-1079 Riga (LV)
(74) Representative: Fortuna, Aleksandra

(56) References cited:
- JP-A- 2002 114 668
- Gordana M. Zdunic: "Comparative chemical analysis and pharmacological profile of extracts of selected species of the genus Hypericum L. Doctoral Dissertation.", , 2012, page 194pp, XP002751159, University of Belgrade Retrieved from the Internet: URL:https://fedorabg.bg.ac.rs/fedora/get/o :5448/bdef:Content/get [retrieved on 2015-11-24]
- L. V. SHATUNOVA: "Flavonoids of Hypericum hirsutum", CHEMISTRY OF NATURAL COMPOUNDS, vol. 14, no. 4, 1979, pages 444-445, XP002751160,
- T. OZEN ET AL.: "TOTAL PHENOL CONTENT OF SOME Hypericum SPECIES GROWING IN TURKEY", CHEMISTRY OF NATURAL COMPOUNDS, vol. 41, no. 2, 2005, pages 232-233, XP002751161,

## Description

### Technical field

This invention relates to cosmetic agents and compositions for skin whitening with skin renewal properties.

### Prior art

High regenerative potential and ecological plasticity of plants has rendered *in vitro* cultivation of plant cells and tissues to become a routine and widely applied technology for industrial application in many fields. Plant *in vitro* culture based technological solutions for active compound production for cosmetics is becoming an environmentally friendly alternative to wild plant harvest or agricultural cultivation [1]. Due to high product standardization and seasonal independence, the process is more amenable to requirements of high quality standards of cosmetic industry. Besides, due to growing demand for organic and natural products of personal care, industry and regulatory institutions face problems related to natural product safety and purity control guaranteeing absence of any chemical or microbial contamination or plant species misidentification related impurities [2]. Plant cultivation *in vitro* excludes any effects of misidentification, environmental pollution, microbial contamination or side effects of intensified agriculture, thus opening a possibility for truly biological and organic ingredient production for cosmetics. Although plant cell cultures are gaining rapidly increasing interest of cosmetic industry, often low yield of target compounds and genetic instability can be problematic for industrial application [3]. Instead, *in vitro* cultivation of differentiated plant tissues ensure high yield of tissue-specific metabolites offering an alternative to cell cultures for *in vitro* culture based production of active compounds [4].

Owing to high content of naphtodianthrones - hypericin, pseudohypericin and flavonoids - hyperoside, quercetin and rutin species of genus *Hypericum* comprise valuable pharmaceutical activity [5]. Although majority of pharmaceutical studies regarding *Hypericum* species are focused on *H.performatum*, other species of the genus -have also been shown to hold a high pharmaceutical potential [6], [7], [8], [9], [35]. Besides, rich phytochemical content of other *Hypericum* species assessed by MS and NMR metabolomics [10] suggests that numerous other species of *Hypericum* appart of *H.perforatum*, might be valuable despite of unexplored pharmaceutical activity. Studies concerning pharmacological *H.hirsutum* have revealed high anti-microbial activity and antioxidative capacity of the species [11], [12]. Chemical characterization of wild *H.hirsutum* has identified high content of valuable target molecules [13], [14] suggesting that *H.hirsutum* might become a valuable source of active compounds.

Kaempferol-7-O-glucoside is described as suitable depigmenting and anti-aging agents and is believed to reduce pigmentation by inhibiting tyrosinase but may also reduce pigmentation by alternate pathways [15].

Chlorogenic acid and 5-Caffeoylquinic acid are the esters formed between caffeic acid and L-quinic acid and are a widespread phenolic compounds. Chlorogenic acid is also widely used in cosmetics contributes to light protection in the sun tan cosmetics, anti-oxidant, smoothes the skin etc., but the effects of Chlorogenic Acid on melanogenesis are still unknown. In [16] the effects of Chlorogenic Acid on cell proliferation, melanin content and tyrosinase of B16 murine melanoma cells is described. Additionally, the enzymatic reactions of Chlorogenic Acid in B16 melanoma cells lytic solution were detected by UV spectrophotometry. It was concluded that Chlorogenic Acid is involved in melanogenesis and affects the activity of tyrosinase. Chlorogenic Acid A can suppress cell proliferation accelerated by 8-MOP. Chlorogenic Acid might be a substrate of melanogenic enzymes in B16 cells. Chlorogenic Acid can suppress melanogenesis in B16 melanoma cells by inhibiting enzymatic oxidation of a diphenol, especially in B16 melanoma cells activated by 8-MOP. Kaempferol-3-O-glucoside has potential to be a moisturising agent. Atif Ali et al. 2013 tried to evaluate the moisturizing effects of cosmetic creams containing extract of Moringaoleifera leaves (Sohajana) by non-invasive bioengineering techniques. It was found that besides other constituents, this plant contains Kaempferol-3-O-glucoside,Chlorogenic acid and 5-Caffeoylquinic acid. The results recommended that the assessed outcome was very well accepted, and improved skin hydration and skin barrier function by the test product. There was a significant decrease in skin transepidermal water loss.

Umbelliferone is widespread coumarin used in cosmetics as fragrance, skin-whitening and anti-hyperpigmentation product. Umbelliferone absorbs ultraviolet light strongly at several wavelengths (300, 305, 325 nm) and is used in sunscreens. It is also used as an antioxidant with minimal toxicity. It also has anti-inflammatory activity as it decreases lipid peroxidation. Thus, Umbelliferone is a phytochemical with sun-blocking, antioxidant and anti-inflammatory properties. [27].

High antioxidative potential, anti-inflammatory and anti-microbial activity and wound healing properties makes a related species *Hypericum* species attractive for development of cosmetical and pharmaceutical products for topical application (Wölfle et al. 2014). Wound healing and anti-inflammatory properties of *H.perforatum* have mainly been attributed to high content of hyperforin and hypericin [33]. Presence of hyperforin and hypericin has also been detected in extracts of wild *H.hirsutum* [13, 14, 29]. Numerous cosmetic formulations containing *H.perforatum* derived ingredients have been described by patent applications WO2012033422 A1, WO2010043346 A1, US7166310 B2, EP1906978 B1, WO2005053720 A1, US7357950 B2, EP2364713 B1, WO2006053415A1). Despite the fact that phytochemical analyses have demonstrated presence of valuable compounds and anti-microbial and antiradical activity of *H.hirsutum,* application of *H.hirsutum* derived ingredients in cosmetic formulations has not been described yet.

Chemical composition and pharmacological properties of species related to *H.perforatum* have been shown to vary significantly depending on environmental growth conditions [17] and geographical origin [7] suggesting that high biological variability might impose problems for industrial application of field grown or wild plant material. Therefore *in vitro* based production of *Hypericaceae* derived active ingredients would have significant advantages with regard to material uniformity, constant chemical composition and thus reliable pharmaceutical performance. Due to high pharmaceutical value, various protocols have previously been proposed for *in vitro* cultivation of species from genus *Hypericum,* including specific protocols for *in vitro* cultivation of *H.hirsutum* [8]. Phytochemical content analyses of *in vitro* derived material in comparison to wild plants of different *Hypericum* species have revealed the capacity of *in vitro* culture systems to produce comparable yield of main target molecules [9]. *In vitro* culture derived *H.hirsutum* plant comprise considerable amount of hypericin and hyperforin [8] supporting the idea of *in vitro* culture based production of *H.hirsutum* active ingredients for application in cosmetics.

Use of *in vitro* plant cultures is a growing trend in cosmetic industry. Use of active ingredients derived from different *in vitro* plant cultures have been described is several patents (US6224850B1, WO2009151302A2, WO2013102882A2, WO2015001524A2, WO2013124540 A1, EP2355782 A2, WO2010067212 A2, EP2324818 A1) and scientific publications (e.g., [3], [28], [32], [34]). Among species cultured *in vitro* for cosmetic use are apple (Malusdomestica), edelweiss (*Leontopodiumalpinum*), grape (*Vitisvinifera*), echinacea (*Echinacea angustifolia*) and many others [20].

### Disclosure of the invention

The present invention relates primarily to production of skin whitening and regenerating active cosmetic ingredients and use of above mentioned ingredients in skin whitening cosmetic formulations. More specifically, use of *in vitro* cultivated *H.hirsutum* for production of extracts for inclusion in skin whitening cosmetic formulations is proposed.

The object of the invention is the skin active extract from *in vitro* cultivated *Hypericum hirsutum.* The dominant isolated constituents of *H.hirsutum* extracts are flavonoids and phenolic acids. The main constituents extracted and separated from *H.hirsutum* ethanolic-aqueous extract are Kaempferol-3-O-glucoside (known as astragalin), Kaempferol-7-O-glucoside (known as populnin), Fisetin, Chlorogenic acid, 3-Caffeoylquinic acid and Umbelliferone. The proposed isolated extract is characterized by antiradical activity and high total phenolic compound content. The extract has the ability to inhibit tyrosinase, the key enzyme in melanin formation and expression of genes related to melanin synthesis such as MLANA (a protein antigen that is found on the surface of melanocytes) and PMEL (premelanosome protein). Extract exhibits inhibitory effect on proliferation of melanocytes and at the same time is not highly cytotoxic in concentration range of 1 -3 %. The proposed concentration range for use in skin whitening cosmetic formulations is 0.5-2% (v/v).

A skin whitening cosmetic active ingredient, being an extract isolated from *in vitro* cultivated *Hypericum hirsutum,* is proposed. Extract is obtained by 50% ethanol extraction, from the plant material derived from *in vitro* shoot cultures with no growth regulators added. The offered ingredient contains Kaempferol-3-O-glucoside, Kaempferol-7-O-glucoside, Fisetin, Chlorogenic acid, 3-Caffeoylquinic acid and Umbelliferone.

The extract is characterized by high total phenolic compound content and high antiradical activity, attributing regenerative properties to the cosmetic composition and protecting from unfavorable external skin ageing-promoting agents . The extract is further characterized by capability to suppress proliferation of melanocytes by 15% during the first 96 h of incubation in case of 1% extract and by 35% of 2% extract and more than 50% in case of 3 and 4 % extract.

### Brief Description of Drawings

Fig. 1 - LC-ESI-TOF MS chromatogram of the ethanolic-aqueous extractof *H.hirsutum.*
Fig. 2 - LC-ESI-TOF MS EIC chromatograms of the **A**-Astragalin and Kaempferol-7-O-glucoside, **B**-Chlorogenic acid and 5-Caffeoylquinic acidin *H.hirsutum.*
Fig. 3 - diagram, showing effect of *H.hirsutum* extract on melanocyte (FM 55 cell line) proliferation.

### Establishment of in vitro shoot cultures

Seeds of *Hypericum hirsutum* were provided by Botanical garden of University of Latvia. Seeds were surface sterilized for 10 min with commercial bleach containing 5% w/w NaOCl, rinsed 3 times with deionized distilled sterile water and planted on MS medium containing 3% (w/v) sucrose and solidified with 0.75% (w/v) agarose. No growth regulators were added. pH was adjusted with 1M NaOH to 5.7 prior to autoclaving 20 min at 120°C. Seeds were germinated at 23°C in 16h/8h light/dark period. Sufficiently developed shoots were subsequently divided in fragments which were then used as explants for culture propagation.

### Extraction of plant material and analysis of phytochemical contents

For the phytochemical analysis 12 week old 2nd passage *in vitro* grown plants were collected, air-dried at 25°C until constant weight and disintegrated with mortar and pestle. 40 mg of ground plant material were extracted with 1 ml 50 % ethanol by vortexing at 2200 rpm/min for one minute. The extract was cleared by centrifugation (4,000 g × 10 min) at room temperature. The resulting supernatant was filtered with 0.2 *µ*m filters, to remove particulate and other suspended solid matter.

Chromatographic analyses were performed on a modular LC system, Agilent *1290 Infinity* series (Agilent Technologies, Germany). LC separations were achieved by using an *XTerra MS C18 3.5 µm*, *2.1x150mm* (Waters). Elution solvents consist of 0.1% formic acid in acetonitrile and 0.1% formic acid in water in gradient mode. The injection volume was 10 *µ*L. The high resolution mass spectra (HRMS) were taken, respectively, on an *Agilent 6230 TOF LC*/*MS* (Agilent Technologies, Germany) with electrospray ionization (ESI). Source was used with the following parameters: positive ionization mode, drying gas 10 mL/min and 325°C, fragmentor ionization 130 V. Internal reference mass 121.050873 m/z and 922.009798 m/z for all sample analyses were used. Data analyses were performed using MassHunter version B05.00 software (Agilent Technologies). The filtered samples were injected into LC system. A calibration curve of *gallic acid* 5 standard solutions ranging from 0.01 - 25 µg/ml were constructed by plotting the average peak area against concentration, and a regression equation was computed. Standard solution at each concentration was injected three times and the corresponding peak areas were recorded. The relative standard deviation was determined to be less than 0.5%. The obtained calibration curve showed linearity of correlation coefficient (R²) in the concentration range 0.998. Coefficient of determination (R²) was calculated using Microsoft *Excel 2013*, p<0.001. Peak confirmations were made by a high resolution mass spectral evaluation via MassHunter B05.00 software (Agilent Technologies).

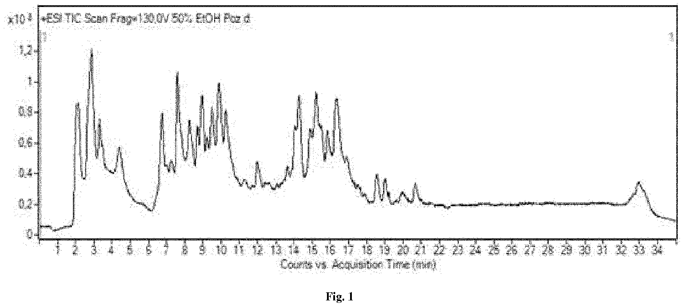

The *H.hirsutum* ethanolic-aqueous extract was analysed using liquid chromatography electrospray ionization time of flight mass spectrometry (LC-ESI-TOF MS). Figure 1 shows Total Ion Current (TIC) chromatogram of all separated compounds.
Identification of separated compounds was based on the search for [M+H]⁺ ions, using extracted ion mass chromatograms. In total 33 constituents were identified representing total amount 25.7 mg/1g DW of *H.hirsutum* ethanolic-aqueous extract. All identified compounds, their retention times; amount and molecular weight are summarized in table 1.

**Table 1**

| **Phytochemical compounds extracted from *in vitro* samples of *H.hirsutum*** | | | | |
|---|---|---|---|---|
| No | **Compound** | **t_{R}, min** | **m/z found** | **C µg/1gDW** |
| 1 | Kaempferol -3-O-glucoside | 8,93 | 449,1078 | 8810,4 |
| 2 | Fisetin | 8,96 | 287,055 | 4242,6 |
| 3 | Chlorogenic acid | 7,76 | 355,1024 | 2160,7 |
| 4 | Umbelliferone | 7,76 | 163,0391 | 1988,1 |
| 5 | 3-Caffeoylquinic acid | 8,28 | 355,1024 | 1754,9 |
| 6 | Kaempferol -7-O-glucoside | 7,61 | 449,1078 | 1352,1 |
| 7 | Cyanidin | 8,96 | 288,0628 | 747,0 |
| 8 | Vanillic acid | 7,26 | 169,0495 | 741,5 |
| 9 | Vapiprost | 15,00 | 478,2952 | 737,5 |
| 10 | Coumarin | 8,26 | 147,0441 | 432,5 |
| 11 | p-Hydroxybenzoic acid | 8,69 | 139,0390 | 431,5 |
| 12 | Isovitexin | 9,41 | 433,1116 | 402,9 |
| 13 | Quinic acid | 3,04 | 193,0708 | 317,5 |
| 14 | Cynarin | 10,71 | 517,1341 | 284,7 |
| 15 | Gallocatechol | 7,98 | 307,0812 | 188,1 |
| 16 | Eriodiktiol | 7,43 | 289,0707 | 149,4 |
| 17 | Moroctic acid | 14,96 | 277,2177 | 123,2 |
| 18 | o-Coumaric acid | 4,71 | 165,0546 | 111,3 |
| 19 | Quercetin-3-O-glucoside | 8,43 | 465,1017 | 107,5 |
| 20 | Mangiferitin | 3,23 | 261,0394 | 91,6 |
| 21 | Ferulic acid | 7,96 | 195,0652 | 89,2 |
| 22 | Niacinamide | 3,21 | 123,0553 | 85,6 |
| 23 | Syringic acid | 7,44 | 199,0601 | 73,0 |
| 24 | Quercetin | 8,41 | 303,0499 | 60,3 |
| 25 | Pirogallic acid | 6,99 | 127,0390 | 39,3 |
| 26 | Pinoresinol | 12,20 | 359,1489 | 33,1 |
| 27 | Quercetin glucuronide | 9,60 | 479,0825 | 27,5 |
| 28 | Hesperetin | 8,03 | 303,0863 | 25,7 |
| 29 | p-Coumaric acid | 3,46 | 165,0546 | 22,3 |
| 30 | Aloesin | 8,36 | 395,1357 | 19,1 |
| 31 | Hyperforin | 22,33 | 537,3938 | 11,6 |
| 32 | Spinochrome A | 14,68 | 265,1465 | 9,3 |
| 33 | Glycitein 7-O-glucoside | 10,85 | 447,1282 | 6,3 |

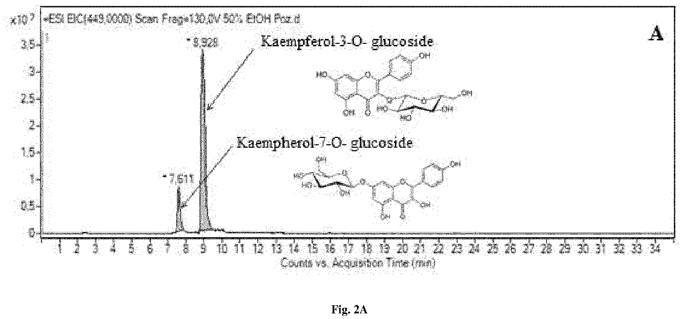

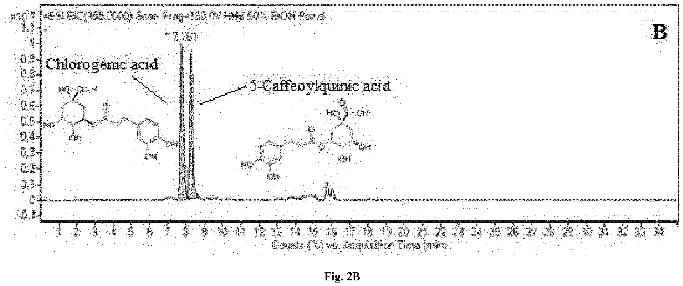

The main constituents extracted and separated from *H.hirsutum* ethanolic-aqueous extract are Kaempferol-3-O-glucoside (known as astragalin), Kaempferol-7-O-glucoside (known as populnin), Fisetin, Chlorogenic acid, 3-Caffeoylquinic acid and Umbelliferone. Figure 2 shows LC-ESI-TOF MS extracted ion chromatograms (EIC) of some separated and identified constituents. The dominant isolated constituents of *H.hirsutum* extracts are flavonoids and phenolic acids.

### Total phenolic content (TPC) and anti-radical activity (ARA) of in vitro shoot culture derived H.hirsutum extracts.

TPC and ARA were determined in aqueous-alcoholic (40 mg DW/ml 50% EtOH) extract of *H.hirsutum* essentially as described by Herald et al. 2012.

*In vitro* shoot culture derived *H.hirsutum* showed high total phenolic compound content and significant antiradical activity. TPC of *H.hirsutum* ethanol-water extract was 62 ± 16 mg GAE/ g DW. Whereas ARA of the tested extract was equal to 319 ± 3 mg Ascorbic acid equivalents/ g DW (83 ± 0.9% 150µM DPPH scavenging for 1% (v/v) extract).

### Tyrosinase inhibiting activity of in vitro shoot culture derived H.hirsutum extracts.

Tyrosinase inhibiting activity was assayed according to methods described by Massamoto et al. 2003. Dry mass of *H.hirsutum* extract was resuspended in water and 5 µl aliquots of the solution were mixed with 90 µl of 0,05M potassium phosphate buffer (pH 6,8) and 50 ml of 1,25 mM L-DOPA solution. After incubation for 10 minutes at room temperature 5 µl of 1000 u/ml mushroom tyrosinase was added, and solution monitored for dopachrome formation by optical density measurement at 475 nm. Kojic acid was used as a positive control. IC50 values where calculated.

*H.hirsutum* extract shows remarkable inhibitory activity - at concentration 60 mg/ml extract inhibits 73,09± 25,08% of the enzyme activity, at concentration 30 mg/ml the inhibitory activity is 49,28 ± 23,05%. IC50 of the extract is 32,93± 14,94 mg/ml.

The ability to inhibit the key enzyme in melanin formation indicates the high potential of the *H.hirsutum in vitro* culture derived extract for skin whitening.

### Effect on proliferation of melanocytes (FM 55 cell line)

For time-dependent cell response profiling cells were grown in 96 well plate (Sarstedt) and analyzed with IncuCyte ZOOM (Essen BioScience). Cells were seeded in triplicates in medium (DMEM /10% FBS and antibiotics) at density 2x10³ cells per well. The 96 well plate containing the cells was placed on the reader in the incubator at 37°C, 5% CO₂ for continuous recording of confluence. After 48 hours *H.hirsutum* extract (40 mg/ml) or ethanol as solvent control was added to growth medium in concentrations ranging from 0.25% - 4% (v/v of medium). Cells were continuously grown for 96 hours and confluence recorded every hour. Experiment was done on three different melanocyte passages.

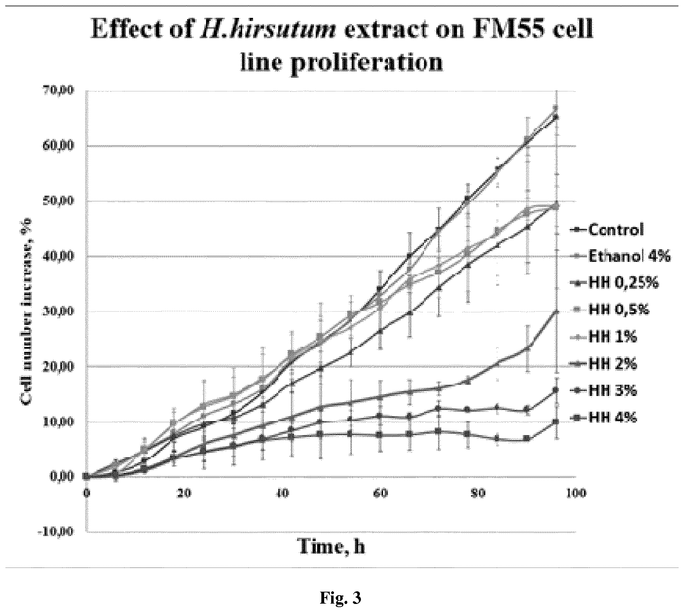

1% (v/v of media) extract from *in vitro* cultivated *H.hirsutum* showed approximately 15% reduction of cell proliferation, but in case of 2% extract in cell growth media- almost 35% reduction, while the 3% and 4 % almost stopped cell proliferation (Figure 3).

Results indicate that *H.hirsutum* extract effectively reduces proliferation of melanocytes and thereby can be an effective ingredient of whitening cosmetics..

### Effect of H.hirsutum extract on melanin synthesis-related gene expression

RNA from FM 55 cells was extracted using TRIzol reagent (Invitrogen) according to manufacturer's recommendations. First strand cDNA synthesis was performed on 0.5 µg total RNA using RevertAid™ M-MuLVReverse Transcriptase (Thermo Scientific) and quality assay for cDNA synthesis was PCR reaction (25 cycles) with human GAPDH primers. Gene expression levels were quantified by real-time PCR with ABI Prism 7300 (Applied Biosystems) using Maxima SYBR Green/ROX qPCR Master mix (Thermo Scientific). In brief, 20 µl reaction mixture contained 25 ng single stranded cDNA, gene-specific forward and reverse primers (final concentration 0.4 µM), and 10 µl SYBR Green Master mix. Reaction conditions were as follows: initial activation step at 95°C for 5 min, 40 cycles of melting at 95°C for 15 sec and annealing/extension at 60°C for 30 sec, data were collected in additional 72°C 30 sec cycle. Samples were assayed in duplicates. Relative fold-changes were quantified using the comparative CT (2^{-ΔΔCT}) method (Livak and Schmittgen 2001). Data were normalized to the housekeeping gene GAPDH mRNA expression level. Expression levels were calculated comparing control with extract or solvent treated samples.

Presence of 2% *in vitro* shoot culture derived *H.hirsutum* extract in FM 55 cell growth media decreased expression of tyrosinase gene (TYR) (enzyme for controlling the production of melanin) for more than a half (49,3 %) compared with the non-treated cells. The same decreasing effect was observed on two other with melanin synthesis-related gene expression - MLANA and PMEL (22,6 % and 38,6%) compared with control which is considered as 100% expression. Inhibition of the gene expression was dose dependent. There was no remarkable inhibitory activity of solvent (2% of 50% ethanol solution) on named gene expression.

Decreasing of melanin synthesis-related gene expression shows the ability of *in vitro* shoot culture derived *H.hirsutum* extracts to reduce skin pigmentation by inhibition of melanin synthesis.

### Possible cosmetic compositions containing extract isolated from in vitro cultivated H.hirsutum.

### Example 1 - regenerating facial cream

| | | |
|---|---|---|
| a) | Aqua | 54,70% |
| b) | Vitis vinifera (Grape) Seed Oil | 20% |
| | Caprylic/capric triglycerides | 10% |
| | Titanium dioxide | 6% |
| | Olivem-100 (Cetearyl Olivate, Sorbitan Olivate) | 4% |
| | Cetyl alcohol | 2% |
| c) | *H.hirsutum* extract from *in vitro* cultures | 2,00% |
| | Tocopherol acetate | 0,5% |
| | Potassium sorbate | 0,4% |
| | Gluconolactone, Sodium Benzoate | 0,4% |
| | | |

Ingredient a) is heated to approximately 65°C b) is likewise heated to approximately 65 °C till emulsifying waxes are completely melted, and then added while vigorously stirring to mixture a) to form emulsion. Stirring is continued until the cream has cooled down to approximately 35 °C. Then mixture c) is added while stirring and the cream is homogenized.

### Example 2 - regenerating serum for periocular skin

| | | |
|---|---|---|
| a) | Aqua | 73,17% |
| b) | Squalane | 10% |
| | Coco-caprylate | 10% |
| | Olivem-100 (CetearylOlivate, SorbitanOlivate) | 3% |
| | Cetylalcohol | 2% |
| c) | *H.hirsutum* extract from *in vitro* cultures | 0.5% |
| | Tocopherolacetate | 0,5% |
| | Potassiums orbate | 0,4% |
| | Gluconolactone, Sodium Benzoate | 0,4% |
| | Ubiquinone | 0,03% |
| | | |

Ingredient a) is heated to approximately 65°C and mixture b) is likewise heated to approximately 65 °C till emulsifying waxes are completely melted, and then added while vigorously stirring to mixture a) to form emulsion. Stirring is continued until the formulation has cooled down to approximately 35 °C. Then mixture c) is added while stirring and formulation is homogenized.

### References

1. AbouZid, S. 2014. Yield improvement strategies for the production of secondary metabolites in plant tissue culture: silymarin from Silybummarianum tissue culture. Natural product research 28.23: 2102 - 2110
2. Ali, A., Akhtar, N., Khan, M. S., Rasool, F., Iqbal, F. M., Khan, M. T. and Elahi, E. 2013. Moisturizing effect of cream containing Moringa oleifera (Sohajana) leaf extract by biophysical techniques: In vivo evaluation. Journal of Medicinal Plants Research 7(8): 386 - 391
3. Ananga, A., Georgiev, V., Ochieng, J., Phills, B. and Tsolova, V. Production of anthocyanins in grape cell cultures: A potential source of raw material for pharmaceutical, food, and cosmetic industries. INTECH Open Access Publisher, (2013).
4. Antignac, E., Nohynek, G.J., Re, T. ,Clouzeau, J., Toutain, H. 2011. Safety of botanical ingredients in personal care products/cosmetics. Food and Chemical Toxicology 49: 324 - 341
5. Asgarpanah, J. 2012. Phytochemistry, pharmacology and medicinal properties of Hypericum perforatum L. African Journal of Pharmacy and Pharmacology 6: 1387 - 1394
6. Barbulova, A., Apone, F., Colucci, G. 2014. Plant Cell Cultures as Source of Cosmetic Active Ingredients. Cosmetics 1: 94 - 104
7. Bruni, R., Sacchetti, G. 2009. Factors affecting polyphenol biosynthesis in wild and field grown St. John's Wort (Hypericum perforatum L. Hypericaceae/Guttiferae). Molecules 14: 682 - 725
8. Coste, A., Vlase, L., Halmagyi, A., Deliu, C., Coldea, G. 2011. Effects of plant growth regulators and elicitors on production of secondary metabolites in shoot cultures of Hypericumhirsutum and Hypericummaculatum. Plant Cells, Tissues and Organ Cultures 106: 279 - 288
9. Danova, K., Nikolova - Damianova, B., Denev, R., Dimitrov, D. 2012. Influence of vitamins on polyphenolic content, morphological development, and stress response in shoot cultures of Hypericum spp. Plant Cells, Tissues and Organ Cultures 110: 383 - 393
10. Gudž̌̌̌̌ic, B. T., Šmelcerović, A., Dordević, S., Mimica-Dukić, N., Ristić, M. 2007. Essential oil composition of Hypericum hirsutum L. Flavour and fragrance journal 22: 42-43
11. Herald, T.J., Gadgil, P., Tilley, M. 2012. High-throughput micro plate assays for screening flavonoid content and DPPH-scavenging activity in Sorghum bran and flour. Journal of the Science of Food and Agriculture 92: 2326 - 2331
12. Jiang, L., Numonov, S., Bobakulov, K., Kureshi, N.M., Zhao, H., Aisa, H.A. 2015. Phytochemical profiling and evaluation of pharmacological activities of Hypericum scabrum L. Molecules 20: 11257 - 11271
13. Kitanov, G.M. 2001. Hypericin and pseudohypericin in some Hypericum species. Biochemical Systematics and Ecology 29: 171 - 178
14. Kusari, S., Zuhlke, S., Borsch, T., Spiteller, M. 2009. Positive correlations between hypericin and putative precursors detected in the quantitative secondary metabolite spectrum of Hypericum. Phytochemistry 70: 1222 - 1232
15. Li, H.-R., Habasi, M., Xie, L.-Z., Aisa, H.A. 2014. Effect of Chlorogenic Acid on Melanogenesis of B16 Melanoma Cells. Molecules 19: 12940 - 12948.
16. Livak, K. J., and Schmittgen, T. D. 2001. Analysis of relative gene expression data using real-time quantitative PCR and the 2- ΔΔCT method. Methods 25(4): 402 - 408.
17. Marrelli, M., Conforti, F., Toniolo, C., Nicoletti, M., Statti, G., Menichini, F. 2014. Hypericum perforatum: Influences of the habitat on chemical composition, photo-induced cytotoxicity, and antiradical activity. Pharmaceutical Biology 52: 909 - 918
18. Masamoto, Y., Ando, H., Murata, Y., Shimoishi, Y., Tada, M., Takahata, K. 2003. Mushroom tyrosinase inhibitory activity of esculetin isolated from seeds of Euphorbia lathyris L. Bioscience, Biotechnology, and Biochemistry 67: 631 - 4.
19. Meinke, M. C., Schanzer, S., Haag, S.F., Casetti, F., Müller, M.L., Wölfle, U., Kleemann, A., Lademann, J., Schempp C.M. 2012. In vivo photoprotective and anti-inflammatory effect of hyperforin is associated with high antioxidant activity in vitro and ex vivo. European Journal of Pharmaceutics and Biopharmaceutics 81: 346 - 350
20. Moruś, M., Baran, M., Rost-Roszkowska, M., Skotnicka-Graca, U. 2014. Plant stem cells as innovation in cosmetics. Acta Poloniae Pharmaceutica Sep-Oct; 71(5):701 - 7.
*21*. Mukherjee, P.K., Verpoorte, R., Suresh, B. 2000. Evaluation of in-vivo wound healing activity of Hypericum patulum (Family: Hypericaceae) leaf extract on different wound model in rats. Journal of Ethnopharmacology 70: 315 - 321
22. Murthy, H.N., Lee, E.-J., Paek, K.-Y. 2014. Production of secondary metabolites from cell and organ cultures: strategies and approaches for biomass improvement and metabolite accumulation. Plant Cell, Tissue and Organ Cultures 118: 1 - 16
23. Porzel, A., Farag, M.A., Mülbradt, J., Wessjohann, L.A. 2013.Metabolite profiling and fingerprinting of Hypericum species: a comparison of MS and NMR metabolomics. Metabolomics 10: 574 - 588
24. Quassinti, L., Lupidi, G., Maggi, F., Sagratini, G., Papa, F., Vittori, S., Bianco A., Bramucci, M. 2013. Antioxidant and antiproliferative activity of Hypericum hircinum L. subsp. majus (Aiton) N. Robson essential oil. Natural product research 27: 862 - 868.
25. Radulović, N., Stankov-Jovanović, V., Stojanović, G., Šmelcerović, A., Spiteller, M., Asakawa, Y. 2007. Screening of in vitro antimicrobial and antioxidant activity of nine Hypericum species from the Balkans. Food Chemistry 103: 15 - 21
26. Sagratini, G., Ricciutelli, M., Vittori, S., Öztürk, N., Öztürk, Y., Maggi, F. 2008. Phytochemical and antioxidant analysis of eight Hypericum taxa from Central Italy. Fitoterapia 79: 210 - 213
27. Sarkar, R., Arora, P., and Garg, K. V. 2013. Cosmeceuticals for hyperpigmentation: What is available? Journal of cutaneous and aesthetic surgery 6(1): 4
28. Schürch, C., Blum, P., Zülli, F. 2008. Potential of plant cells in culture for cosmetic application. Phytochemistry Reviews 7.3: 599 - 605
29. Smelcerovic, A., Verma, V., Spiteller, M., Ahmad, S. M., Puri, S. C., Qazi, G.N. 2006. Phytochemical analysis and genetic characterization of six Hypericum species from Serbia. Phytochemistry 67: 171 - 177
30. Steingroewer, J., Bley, T., Georgiev, V., Ivanov, I., Lenk, F., Marchev, A., Pavlov, A. 2013. Bioprocessing of differentiated plant in vitro systems. Engineering and Life Sciences 13: 26 - 38
31. Stojanovic, G., Dordevic, A., Smelcerovic, A. 2013. Do other Hypericum species have medical potential as St. John's wort (Hypericum perforatum)? Current medicinal chemistry 20: 2273 - 2295
32. Tito, A., Carola, A., Bimonte, M., Barbulova, A., Arciello, S., de Laurentiis, F., Monoli, I., Hill, J., Gibertoni, S., Colucci, G., Apone, F. 2011. A tomato stem cell extract, containing antioxidant compounds and metal chelating factors, protects skin cells from heavy metal-induced damages. International journal of cosmetic science 33.6: 543 - 552
33. Wölfle, U., Seelinger, G., Schempp, C. M. 2014. Topical application of St. John's Worth (Hypericum perforatum). Planta Medica 80: 109 - 120
34. Wu, J. and Zhong, J. J. 1999. Production of ginseng and its bioactive components in plant cell culture: current technological and applied aspects. Journal of biotechnology 68(2): 89 - 99
35. JP 2002 114 668 A.

## Claims

1. A skin whitening cosmetic active ingredient, being an extract isolated from *in vitro* cultivated *Hypericum hirsutum* by 50% ethanol extraction, wherein said active ingredient is prepared by a process comprising the steps of:
(i) providing *in vitro* grown *Hypericum hirsutum* plants,
(ii) drying said plants until constant weight,
(iii) disintegrating the dried plants,
(iv) extracting ground plant material by 50% ethanol extraction, selecting the weight ratio of plant material to the extractant in the amount of _1:25 (w/v);
(v) clearing and filtering the extract obtained.

2. The ingredient according to claim 1, wherein the *H.hirsutum* plant material for extraction is obtained by propagation of *in vitro* shoot cultures with no growth regulators added.

3. The ingredient according to any one of the preceding claims containing Kaempferol-3-O-glucoside, Kaempferol-7-O-glucoside, Fisetin, Chlorogenic acid, 3-Caffeoylquinic acid and Umbelliferone.

4. Use of the ingredient according to any one of the preceding claims in skin whitening cosmetic formulations at concentrations 0,5-2% (v/v) extract.

## Patentansprüche

1. Kosmetischer Wirkstoff zur Hautaufhellung, der als Extrakt aus *in vitro* kultiviertem *Hypericum hirsutum* durch 50%ige Ethanolextraktion isoliert wird, wobei der Wirkstoff durch ein Verfahren hergestellt wird, das die folgenden Schritte umfasst:
(i) Bereitstellung von *in vitro* kultivierten *Hypericum hirsutum-Pflanzen,*
(ii) Trocknen der Pflanzen bis zur Gewichtskonstanz,
(iii) Auflösen der getrockneten Pflanzen,
(iv) Extrahieren von gemahlenem Pflanzenmaterial durch 50%ige Ethanolextraktion, Auswählen des Gewichtsverhältnisses von Pflanzenmaterial zum Extraktionsmittel in einer Menge von 1:25 (G/V);
(v) Säubern und Filtern des erhaltenen Extrakts.

2. Bestandteil nach Anspruch 1, wobei das *H. hirsutum*-Pflanzenmaterial zur Extraktion durch Vermehrung von *in vitro* kultivierten Sproßkulturen ohne Zusatz von Wachstumsregulatoren gewonnen wird.

3. Bestandteil nach einem der vorhergehenden Ansprüche, enthaltend Kaempferol-3-O-Glucosid, Kaempferol-7-O-Glucosid, Fisetin, Chlorogensäure, 3-Caffeoylchinasäure und Umbelliferon.

4. Verwendung des Bestandteils nach einem der vorhergehenden Ansprüche in hautaufhellenden kosmetischen Formulierungen mit Konzentrationen von 0,5 bis 2 % (v/v) Extrakt.

## Revendications

1. Principe actif cosmétique de blanchiment de la peau, étant un extrait isolé à partir d'*Hypericum hirsutum* cultivé *in vitro* par extraction à l'éthanol à 50 %, dans lequel ledit principe actif est préparé par un procédé comprenant les étapes consistant à :
(i) fournir des plantes d'*Hypericum hirsutum* mises en croissance *in vitro,*
(ii) sécher lesdites plantes jusqu'à un poids constant,
(iii) désintégrer les plantes séchées,
(iv) extraire une matière végétale moulue par extraction à l'éthanol à 50 %, sélectionner le rapport pondéral de la matière végétale à l'agent d'extraction dans la quantité de 1:25 (poids/volume) ;
(v) clarifier et filtrer l'extrait obtenu.

2. Principe actif selon la revendication 1, dans lequel la matière végétale d'*H. hirsutum* pour extraction est obtenue par propagation de cultures de pousses *in vitro* sans régulateurs de croissance ajoutés.

3. Principe actif selon l'une quelconque des revendications précédentes contenant du kaempférol-3-O-glucoside, du kaempférol-7-O-glucoside, de la fisétine, de l'acide chlorogénique, de l'acide 3-cafféoylquinique et de l'ombelliférone.

4. Utilisation du principe actif selon l'une quelconque des revendications précédentes dans des formulations cosmétiques de blanchiment de la peau à des concentrations de 0,5 à 2 % (volume/volume) d'extrait.
